# EUROPEAN PATENT APPLICATION

(11) **EP 4 752 893 A1**
(43) Date of publication of application: **03.06.2026**
(21) Application number: 25218388.4
(22) Date of filing: 25.11.2025
(51) Int. Cl.: G16H 10/65, A61B 5/145, G06F 21/62, G16H 40/63, G16H 40/67, G16H 80/00, G16H 15/00

(54) **A METHOD OF OPERATING AN ELECTRONIC DEVICE AND A METHOD OF OPERATING A TERMINAL CORRESPONDING TO A SENSOR USER**

(30) Priority: 29.11.2024 KR 20240175361
(71) Applicant: I-SENS, INC., Seoul 06646 (KR)
(72) Inventor: JANG, Won Tae, 06646 Seoul (KR); PARK, Kyong Lyol, 06646 Seoul (KR); SEO, A Ri, 06646 Seoul (KR); YOU, Choong Beom, 06646 Seoul (KR); PARK, Wan Joo, 06646 Seoul (KR); OH, Su Kyong, 06646 Seoul (KR); LEE, Jessica, 06646 Seoul (KR); JUNG, Jae Won, 06646 Seoul (KR); LEE, Ha Young, 06646 Seoul (KR)
(74) Representative: BCKIP Part mbB

(57) **Abstract**

Provided is a method of operating an electronic device, the method including identifying a first blind group including at least one sensor user and at least one healthcare provider, based on an input of one or more healthcare providers among the at least one healthcare provider, setting a blind mode for one or more sensor users among the at least one sensor user and based on a request of the one or more healthcare providers, providing the one or more healthcare providers with biometric information on the one or more sensor users for whom the blind mode is set, and based on a request of a third part other than the one or more healthcare providers, provided is the biometric information blurred for the one or more sensor users for whom the blind mode is set.

## Description

### CROSS-REFERENCE TO RELATED APPLICATION(S)

This application claims the benefit of Korean Patent Application No. 10-2024-0175361, filed on November 29, 2024, in the Korean Intellectual Property Office.

### TECHNICAL FIELD OF THE INVENTION

Example embodiments relate to a method and a device for providing biometric information of a sensor user for whom a blind mode is set in response to a request of a healthcare provider who sets the blind mode for a group from which the information to be withheld.

### BACKGROUND

According to example embodiments, a doctor or a physician may monitor glucose (blood sugar) data of a patient in real time from a continuous glucose monitoring system (CGMS). However, when clinical tests or experiments are conducted based on glucose data measured from CGMS, if a patient can check the glucose data of himself/herself, there is a risk that random factors, such as the patient's dietary control, may affect the experiment. Even when the data is blocked for the patient, if the patient can indirectly recognize or check the data through a healthcare platform other than CGMS, there is a risk that random factors, such as the patient's dietary control, may affect the experiment. Therefore, there is a need for the patient's measurement data to be withheld from a third party or entities other than the medical staff conducting the experiment over a certain period of time.

### SUMMARY OF THE INVENTION

An aspect provides an electronic device that is configured to provide biometric information of a sensor user for whom a blind mode is set, in response to a request of a healthcare provider that sets the blind mode for a group from which the information to be withheld.

The technical tasks to be achieved by the present example embodiments are not limited to the technical tasks described above, and other technical tasks may be inferred from the following example embodiments by those skilled in the art.

According to an aspect, there is provided a method of operating an electronic device, the method including identifying a first blind group including at least one sensor user and at least one healthcare provider, based on an input of one or more healthcare providers among the at least one healthcare provider, setting a blind mode for one or more sensor users among the at least one sensor user and based on a request of the one or more healthcare providers, providing the one or more healthcare providers with biometric information on the one or more sensor users for whom the blind mode is set. Based on a request of a third part other than the one or more healthcare providers, provided is the biometric information blurred for the one or more sensor users for whom the blind mode is set.

In the method of operating the electronic device according to an example embodiment, the biometric information may include at least one biometric information related to glucose.

In the method of operating the electronic device according to an example embodiment, further included may be terminating the blind mode according to an option that is set by the one or more healthcare providers included in the first blind group.

In the method of operating the electronic device according to an example embodiment, the identifying the first blind group may include adding another sensor user that is different from the at least one sensor user included in the first blind group.

In the method of operating the electronic device according to an example embodiment, the adding the sensor user may include using at least one option among an option of using an identification code corresponding to the first blind group, an option of loading a sensor user of another group that is different from the first blind group, and an option of adding a sensor user that is identified by using an exclusive use account regardless of membership registration for a service that the electronic device provides.

In the method of operating the electronic device according to an example embodiment, the option of loading a sensor user of another group that is different from the first blind group may correspond to loading a sensor user that is connected through a service provided by the one or more healthcare providers and the electronic device among sensor users of the another group that is different from the first blind group.

In the method of operating the electronic device according to an example embodiment, the identifying the first blind group may further include deleing one or more sensor users for whom the blind mode is not set in the first blind group, from the first blind group.

In the method of operating the electronic device according to an example embodiment, further included may be setting the first blind group as a normal group when no sensor user for which the blind mode is set exists in the first blind group.

In the method of operating the electronic device according to an example embodiment, the providing the one or more healthcare providers with the biometric information may include providing the biometric information in real time by using at least one of a web, an application and a widget.

In the method of operating the electronic device according to an example embodiment, in the setting the blind mode, when a blind mode is set in a second blind group for a sensor user that is also included in the second blind group that is distinguished from the first blind group among the one or more sensor users, blind mode setting may be deactivated in the first blind group for the sensor user that is also included in the second blind group.

According to another aspect, there is provided a method of operating a terminal corresponding to a sensor user, the method including identifying a blind mode that is set based on an input of a healthcare provider included in a first blind group that includes the sensor user, obtaining biometric information of the sensor user and providing information corresponding to the biometric information that is withheld based on the blind mode. The withheld information is provided in response to a request of a third party other than the healthcare provider.

In the method of operating a terminal corresponding to the sensor user according to an example embodiment, further included may be providing the healthcare provider with biometric information that is obtained based on a request of the healthcare provider.

In the method of operating a terminal corresponding to the sensor user according to an example embodiment, the biometric information may include at least one biometric information related to glucose.

In the method of operating a terminal corresponding to the sensor user according to an example embodiment, further included may be providing the sensor user with information that corresponds to the witheld biometric information regardless of a request of the sensor user.

In the method of operating a terminal corresponding to the sensor user according to an example embodiment, further included may be identifying a blind mode that is terminated according to an option that is set by the healthcare provider included in the first blind group, and providing the biometric information to the sensor user in response to termination of the blind mode.

According to another aspect, there is provided a method of operating an electronic device, the method including identifying a first blind group including at least one sensor user and at least one healthcare provider, based on an input of one or more healthcare providers among the at least one healthcare provider, setting a blind mode for one or more sensor users among the at least one sensor user, and with respect to biometric information on the one or more sensor users for whom the blind mode is set, based on a request of a third part other than the one or more healthcare providers, withholding the biometric information and providing the withheld biometric information to the one or more healthcare providers and the third part that is different from the one or more healthcare providers.

Additional aspects of example embodiments will be set forth in part in the description which follows and, in part, will be apparent from the description, or may be learned by practice of the disclosure.

According to example embodiments, it is possible to propose a method of providing biometric information of a sensor user for whom a blind mode is set, in response to a request of a healthcare provider that sets the blind mode for a group to withhold the information, and the biometric information of the patient cannot be accessed except for the healthcare provider.

The effects achievable by the present disclosure are not limited to the effect mentioned above, and other effects not mentioned will be clearly understood by those of ordinary skill in the art to which the present disclosure pertains from the following description.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1 illustrates an electronic device according to an example embodiment;
FIG. 2 is a diagram illustrating a blind group including at least one healthcare provider and/or at least one sensor user according to an example embodiment;
FIG. 3 is a drawing illustrating the process of setting up a blind group according to an example embodiment;
FIG. 4 is a drawing illustrating the process of adding a sensor user according to an example embodiment;
FIG. 5A to FIG. 5C are drawings illustrating a process of starting and terminating a blind mode according to an example embodiment;
FIG. 6A and FIG. 6B are diagrams illustrating information of a sensor user for whom the blind mode is set by a subject that requests the information according to an example embodiment;
FIG. 7 is a drawing for explaining the process of blurring and providing information of a sensor user for whom the blind mode is set according to an example embodiment;
FIG. 8 is a diagram illustrating a process of a sensor user adding a healthcare provider to a blind group through an application according to an example embodiment;
FIG. 9 is a diagram illustrating a process in which biometric information of a sensor user for whom the blind mode is set is identified in an example embodiment;
FIG. 10 is a diagram illustrating a process by which a sensor user identifies his/her biometric information according to an example embodiment;
FIG. 11 illustrates a flowchart of a method of operating an electronic device according to an example embodiment;
FIG. 12 illustrates a flowchart of a method of operating a terminal corresponding to a sensor user according to an example embodiment; and
FIG. 13 illustrates a flowchart of a method of operating an electronic device according to an example embodiment.

### DETAILED DESCRIPTION OF EXEMPLARY EMBODIMENTS

The following example embodiments may be configured by combining components and features of various example embodiments in predetermined forms. Each of the components and features may be considered to be optional unless explicitly stated otherwise. Each of the components and features may be implemented in a form that is not combined with another component or feature. Alternatively, various example embodiments may be constructed by combining some of the components and the features. The sequence of operations described in various example embodiments may be changed. Some components or features of one example embodiment may be included in another example embodiment, or may be replaced with corresponding components or features of another example embodiment.

In descriptions to be given in conjunction with the accompanying drawings, processes or operations that may obscure the gist of various example embodiments are not described, and processes or operations that are apparent to those of ordinary skill in the art are also not described.

Throughout the specification, when a part is described as "comprising or including" a component, it does not exclude another component but may further include another component unless otherwise stated. Furthermore, terms such as "... unit," "... group," and "... module" described in the specification mean a unit that processes at least one function or operation, which may be implemented as hardware, software, or a combination thereof. Moreover, "a," "an," "one," "the," and similar terms thereto may be used in a sense including both singular and the plural forms in the context of describing various example embodiments (especially in the context of the attached claims) unless otherwise indicated herein or clearly contradicted by context.

Hereinafter, various example embodiments will be described in detail with reference to the accompanying drawings. The detailed description to be given below in conjunction with the accompanying drawings is intended to describe various example embodiments, and is not intended to represent the only embodiments.

Furthermore, specific terms used in various example embodiments are provided to help the understanding of the various example embodiments, and the use of these specific terms may be changed to other forms without departing from the technical spirit of the various example embodiments.

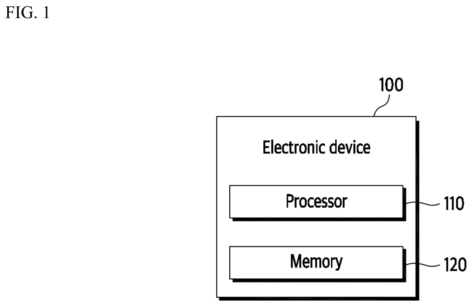

FIG. 1 illustrates an electronic device according to an example embodiment.

Referring to FIG. 1, an electronic device 100 may include a processor 110 and memory 120. The electronic device 100 illustrated in FIG. 1 shows only the components relevant to this example embodiment. Therefore, it will be understood by those skilled in the art that other general components may be included in addition to the components illustrated in FIG. 1, as described in the example embodiment. For example, the electronic device 100 may include a communication device including one or more transceivers, an input part, and an output part. The communication device is a device for performing wired/wireless communication and may communicate with external electronic devices. The external electronic device may be a terminal or a server. Further, communication technologies used by communication devices may include global system for mobile communication (GSM), code division multi access (CDMA), long term evolution (LTE), 5G, wireless LAN (WLAN), Wireless-Fidelity (Wi-Fi), Bluetooth, radio frequency identification (RFID), infrared data association (IrDA), ZigBee, near field communication (NFC) and so on. The input part may be, for example, a traditional keypad or keyboard, a mouse, a microphone that receives voice signals, a camera, or various other input means that detect or receive user input. The output part may be, for example, a display that outputs images, a speaker that outputs sounds, a haptic device that generates vibrations, and various other forms of output means.

The electronic device 100 of FIG. 1 may identify a first blind group that includes at least one sensor user or at least one healthcare provider. Based on the input of one or more healthcare providers among at least one healthcare provider included in the first blind group, the electronic device 100 may set a blind mode for the one or more sensor users among at least one sensor user included in the first blind group. In response to the request of one or more healthcare providers, the electronic device 100 may provide biometric information about the one or more sensor users with the blind mode activated to the one or more healthcare providers.

The processor 110 controls the overall functions of the electronic device 100. For example, the processor 110 controls the electronic device 100 overall by executing programs stored in the memory 120 within the electronic device 100. The processor 110 may be implemented as a central processing unit (CPU), graphics processing unit (GPU), or application processor (AP) within the electronic device 100, but is not limited thereto.

The memory 120 is a hardware that stores various data processed within the electronic device 100, and the memory 120 may store data processed and data to be processed in the electronic device 100. Further, the memory 120 may store applications, drivers and so on to be driven by the electronic device 100. The memory 120 may include random access memory (RAM), such as dynamic random access memory (DRAM), static random access memory (SRAM), read-only memory (ROM), electrically erasable programmable read-only memory (EEPROM), CD-ROM, Blu-ray or other optical disk storage, hard disk drive (HDD), solid state drive (SSD) or flash memory.

The method of operation performed by the electronic device 100 of FIG. 1 may also be implemented by a non-transitory computer-readable storage medium (or non-transitory recording medium) that may be read by a computer for the operation. The method of operation may be implemented as a software module or algorithm, and may be stored on a computer-readable recording medium as computer-readable codes or program instructions executable on the processor 110. Here, the computer-readable recording media include magnetic storage media (for example, read-only memory (ROM), random-access memory (RAM), floppy disks, hard disks and so on) and optical readable media (for example, CD-ROM, digital versatile disc (DVD)). The computer-readable recording media may be distributed across network-connected computer systems, allowing computer-readable code to be stored and executed in a distributed manner. The medium is computer readable, may be stored in memory, and may be executed on the processor 110.

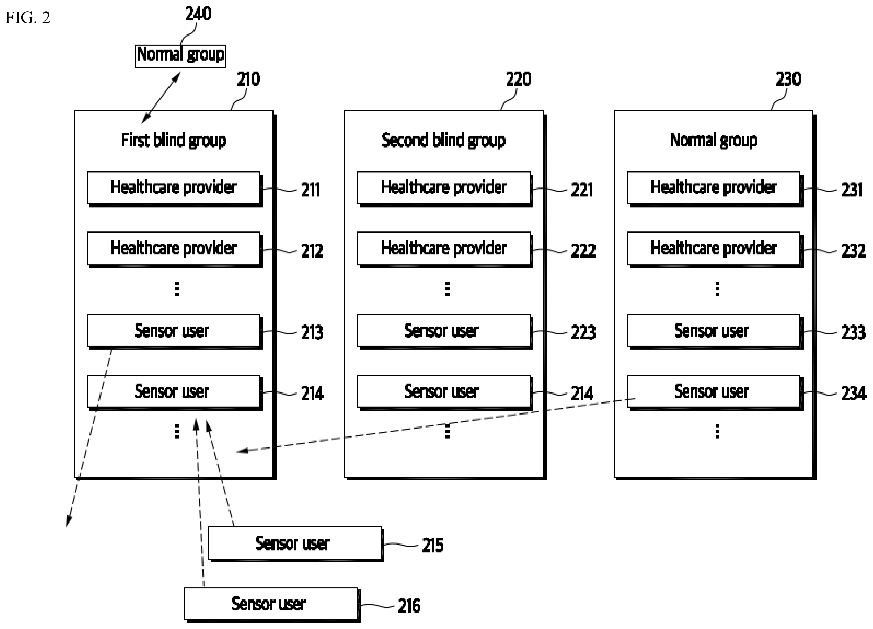

FIG. 2 illustrates a blind group containing at least one healthcare provider and/or at least one sensor user according to an example embodiment. The number of healthcare providers and the number of sensor users illustrated in FIG. 2 pertains to a mere example embodiment, and the scope of rights in the present disclosure is not construed as being limited thereby.

With regard to FIG. 2, example embodiments are described based on a first blind group 210, but the example embodiments may be applied to other blind groups as well, and thus explanations regarding overlapping descriptions are omitted.

Referring to FIG. 2, a normal group 240 may be created that includes at least one healthcare provider (a healthcare provider 211 and a healthcare provider 212) and at least one sensor user (a sensor user 213 and a sensor user 214). The normal group 240 may be set to the first blind group 210 by setting.

Alternatively, a second blind group 220 may be created that includes at least one healthcare provider (a healthcare provider 221 and a healthcare provider 222) and/or at least one sensor user (a sensor user 223 and the sensor user 214). Further, a normal group 230 may be created that includes at least one healthcare provider (a healthcare provider 231 and a healthcare provider 232) and/or at least one sensor user (a sensor user 233 and a sensor user 234).

A blind group and a normal group may not be fixed by a single setting, and depending on the settings, the normal group may be changed to a blind group, and the blind group may be changed to a normal group.

Sensor users and healthcare providers may be included in various normal and blind groups, in addition to the normal and blind groups described in FIG. 2. For example, unlike in FIG. 2, the healthcare provider 211 may be included in another blind group or a normal group in addition to the first blind group 210.

One or more healthcare providers (e.g., the healthcare provider 211) in the first blind group 210 may set a blind mode for one or more sensor users in the first blind group 210. The healthcare provider 211 may request the blind mode approval for the sensor user (e.g., the sensor user 213) to set the blind mode, and the blind mode may be set with the approval for the sensor user 213. When the sensor user 214 is included in both the first blind group 210 and the second blind group 220 as illustrated in FIG. 2, the blind mode may be set for the sensor user 214 in only one blind group, and setting the blind mode multiple times for a single sensor user may be restricted. For example, when the blind mode is set for the sensor user 214 in the second blind group 220, the blind mode settings may be restricted for the sensor user 214 of the first blind group 210.

Referring to FIG. 2, when the blind mode is set for one or more sensor users (e.g. the sensor user 213) of the first blind group 210, biometric information of the sensor user 213 with the blind mode set may be transmitted in real time to the healthcare provider 211 with the blind mode set. Here, the biometric information may include glucose-related biometric information such as average glucose, glucose management indicator (GMI), coefficient of variation, and ambulatory glucose profile (AGP).

Even if there is a request for biometric information of the sensor user 213 from a third party other than the healthcare provider 211 who sets the blind mode, while the blind mode is set, the biometric information of the sensor user 213 may not be provided to any third party other than the healthcare provider 211 who set the blind mode. Meanwhile, the biometric information of the sensor user 213 may be provided to other healthcare providers (e.g., the healthcare provider 212) in the first blind group 210 that includes the healthcare provider 211 that set the blind mode. A third party other than the healthcare provider 211 may include another healthcare provider that is different from the healthcare provider 211 who sets the blind mode and/or a sensor user. For example, even if the biometric information for the sensor user 213 is provided, the biometric information may not be output to other healthcare providers. Alternatively, even if there is a request for biometric information of the sensor user 213 from a healthcare provider other than the healthcare provider 211 that sets the blind mode, the biometric information of the sensor user 213 may be blurred and provided to the healthcare provider that is different from the healthcare provider 211. In other words, even if a request is received from a third party other than the healthcare provider 211 who sets the blind mode, the biometric information of the sensor user 213 may not be provided to the third party, and even if the biometric information of the sensor user 213 is provided, the biometric information of the sensor user 213 may be displayed in a blurred state on a terminal or device of the third party that is different from the healthcare provider 211 that sets the blind mode. The sensor user 213 may need to use a specific sensor to set the blind mode. Here, the specific sensor may be a professional sensor provided by healthcare providers, and when the professional sensor is not used, even if the blind mode is set for the sensor user 213, the blind mode may not be applied to the sensor user 213.

Further, even if there is a request from the sensor user himself/herself for whom the blind mode is set, the biometric information of the sensor user 213 for whom the blind mode is set may not be provided to the sensor user 213 other than the healthcare provider 211 who sets the blind mode. Alternatively, even if the biometric information is provided to a terminal of the sensor user 213, the biometric information may be displayed in a blurred state.

Meanwhile, the blind mode may be terminated by an option specified by the healthcare provider who sets the blind mode. For example, the blind mode that is set for one or more sensor users of the first blind group 210 may be terminated by an option specified by the healthcare provider that sets up the blind mode. Here, the specified option may include manual termination, period expiration, and sensor expiration. The manual termination may correspond to an option by which the blind mode is arbitrarily terminated by an input from the healthcare provider who sets the blind mode, the period expiration may correspond an option by which the blind mode is terminated when the period that is specified when the healthcare provider set up the blind mode expires, and the sensor expiration may correspond to an option by which the blind mode ends when the usage period of the sensor worn by the sensor user expires.

After the first blind group 210 is created, other sensor users may be added to the first blind group 210. For example, an identification code corresponding to the first blind group 210 is generated, and a sensor user 215 that received the identification code may be added to the first blind group 210 based on the identification code. Alternatively, a sensor user 216 may be added to the first blind group 210 by using an exclusive use account regardless of membership in the service provided by the electronic device. Alternatively, if the sensor user 234 of another group (the normal group 230) connected through the service provided by the healthcare provider 211 and electronic device is identified, the healthcare provider 211 may add the sensor user 234 to the first blind group 210 by retrieving information about the sensor user 234.

Unlike adding a sensor user, the healthcare provider 211 may delete the sensor user 213 belonging to the first blind group 210. However, while the blind mode is set for the sensor user 213, the sensor user 213 may not be removed from the first blind group 210.

By changing the settings, the first blind group 210 may be changed to the normal group 240. If there is the sensor user 213 with the blind mode set in the first blind group 210, while the blind mode is in the process, changing the setting from the first blind group 210 to the normal group 240 may be restricted.

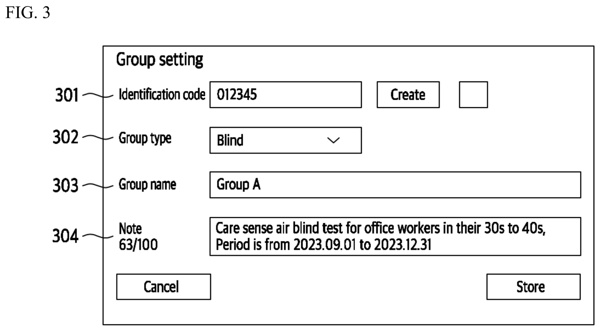

FIG. 3 is a drawing illustrating the process of setting up a blind group according to an example embodiment.

Referring to FIG. 3, a healthcare provider may manage a group based on the web. Here, an identification code in a first area 301 is the identification code corresponding to the group created by the healthcare provider, and may be used in the process of adding sensor users to the created group. The healthcare provider may determine whether a group is set to either a normal group or a blind group through the group type in a second area 302. However, while the blind mode is set for one or more sensor users already in the group, changing the group type of the group to a normal group through the second area 302 may be restricted. Further, a group name in a third area 303 may correspond to the name that the healthcare provider refers to in order to distinguish it from other groups, and the notes in a fourth area 304 may correspond to the characteristics of that group.

In addition there to, the healthcare provider may change various settings to set up the blind mode, such as deleting a group, adding or deleting a sensor user and inviting or deleting a healthcare provider based on the web.

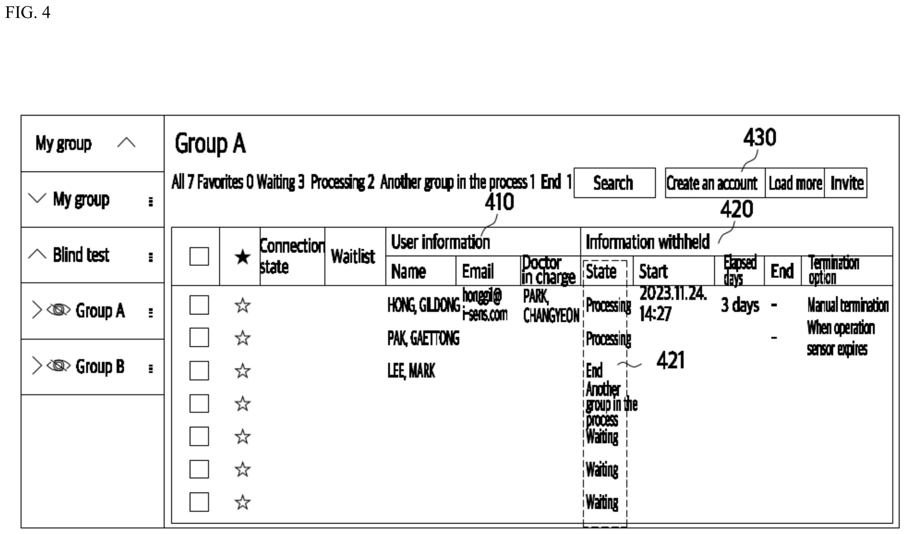

FIG. 4 is a drawing illustrating the process of adding a sensor user according to an example embodiment.

Referring to FIG. 4, identified is the state of Group A. For example, a healthcare provider included in Group A may check information of sensor users included in Group A through a fifth area 410, and check blurred information of each sensor user through a sixth area 420. The blurred information may be information such as blur processing state, processing start date, elapsed days, termination state, and way to terminate for each sensor user. Here, a seventh area 421 may be information contained in the sixth area 420, and the healthcare provider included in Group A may check information about the blur state for each sensor user through the seventh area 421. For example, if the description of the seventh area 421 shows "Processing," it may be that a blind mode is currently applied and in the process, if the description shows "END," the blind mode is ended after the blind mode was applied, if the description shows "Another group in the process," the blind mode is currently being applied in that another group, and if the description shows "Waiting," the blind mode is not applied. In addition thereto, through a screen like FIG. 4, the healthcare provider may check and manage various state information of Group A.

The healthcare provider included in Group A may add sensor users to Group A through an eighth area 430. In an example embodiment, the healthcare provider included in Group A may include the healthcare provider that created Group A and the healthcare provider that is added to Group A. In an example embodiment, even sensor users that are not registered through the "Create an account" icon for the service provided by the electronic device may be added to Group A by creating an account for blind mode exclusive use. Alternatively, at least some of the sensor users already connected to the healthcare provider may be added to Group A via the "Load more" icon. Sensor users who are already connected to the healthcare provider may correspond to sensor users of whom the healthcare provider can request biometric information. For example, the electronic device 100 may connect healthcare providers and patients managed by a physician corresponding to the healthcare provider, i.e. sensor users, through the services provided by the electronic device 100. The healthcare provider may also share the identification code corresponding to Group A with sensor users in various ways, including through the "Invite" icon, and the sensor users may be added to Group A based on their identification code.

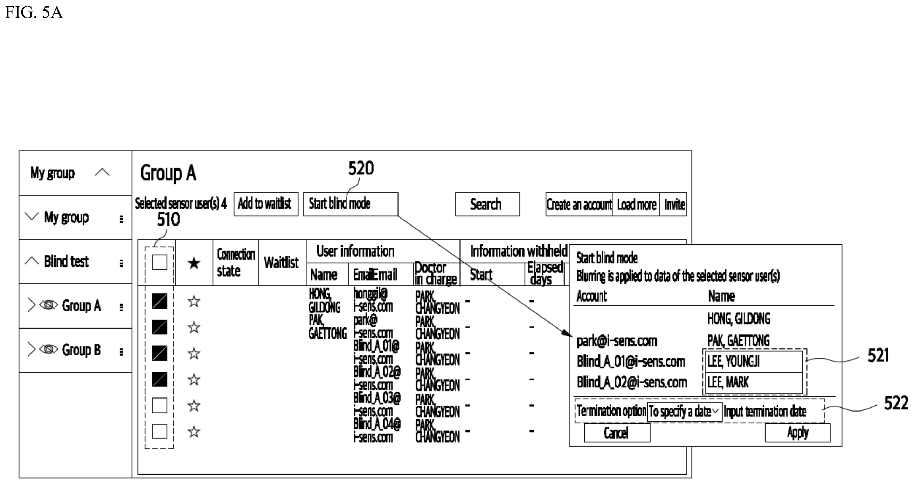

FIG. 5A to FIG. 5C are drawings illustrating a process of starting and ending a blind mode according to an example embodiment.

Referring to FIG. 5A, the healthcare provider in Group A may check the blind mode setting process for sensor users in Group A. The healthcare provider in Group A may set the blind mode for at least one sensor user in Group A. For example, the healthcare provider may select at least one sensor user to set the blind mode via a ninth area 510, and may set the blind mode for at least one selected sensor user via the "Start blind mode" icon in a tenth area 520. Here, in the case of a sensor user added based on an exclusive use account through the "Create an account" icon in FIG. 4 mentioned above, the healthcare provider may enter the sensor user's name through an eleventh area 521. Here, a user added to the group by any option other than the "Create an account" icon in FIG. 4 may enter a nickname instead of his/her real name, and the nickname may be displayed in the user information. However, unlike this, for users added via the "Create an account" icon, the healthcare provider may enter a real name, not a nickname, in the eleventh area 521.

Further, when setting the blind mode, the healthcare provider may preset the way to terminate the blind mode via a twelfth area 522. With regard to the way of terminating the blind mode, the contents described in the present disclosure may be applied.

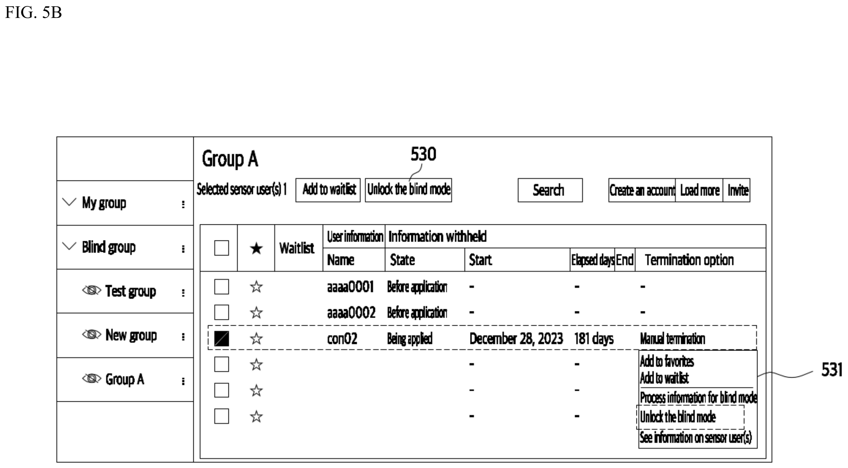

Referring to FIG. 5B, the healthcare provider may monitor the blind mode termination process for sensor users in the blind group for which the blind mode is set. Group A healthcare provider may terminate the blind mode for users in Group A for whom the blind mode is set. For example, the healthcare provider may choose which sensor user of which the blind mode to be terminated, and terminate the blind mode for sensor users who selected the blind mode via the "Off the blind mode" icon in a thirteenth area 530.

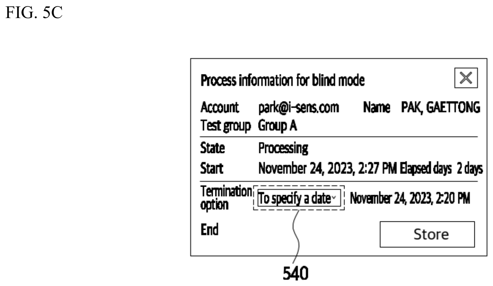

Referring to FIG. 5C, identified is the process information of the set blind mode. The process information of the set blind mode may be identified through a fourteenth area 531 of FIG. 5B. The healthcare provider may change the way to terminate the blind mode through a fifteenth area 540. For example, the healthcare provider may change the way to terminate the blind mode by specifying a termination date, as shown in FIG. 5C, or may change the way to terminate the blind mode in order for the blind mode to be terminated when a sensor usage period is expired or with a method of manual termination, unlike FIG. 5C.

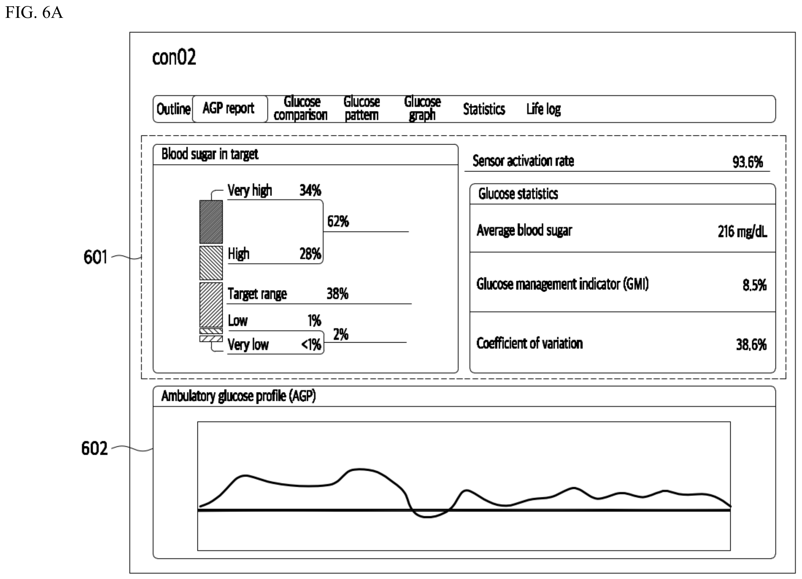

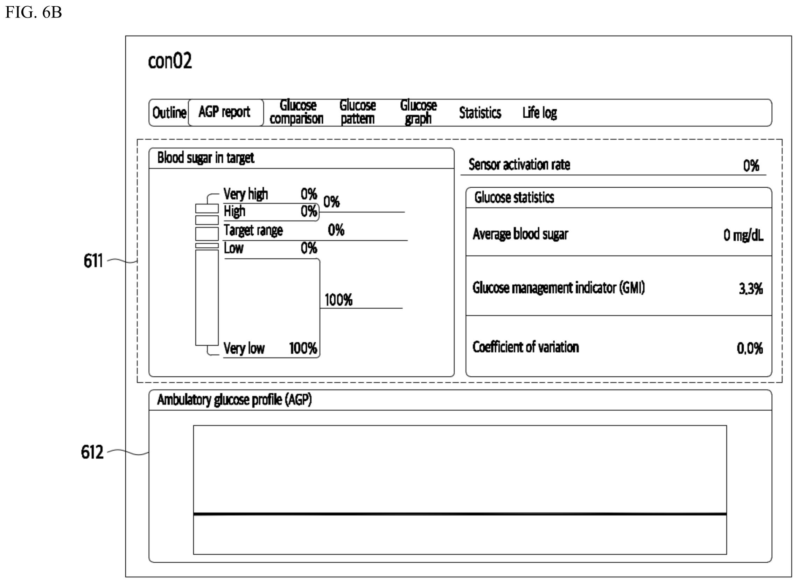

At least one of FIG. 6A and FIG. 6B is a diagram illustrating information of a sensor user for whom the blind mode is set by a subject that requests the information according to an example embodiment.

Referring to FIG. 6A, when there is a request of a healthcare provider that sets the blind mode with respect to a sensor user for whom the blind mode is set, the electronic device 100 provides data related to the sensor user's glucose-related biometric information through a sixteenth area 601, and provide AGP, which is the glucose change trend of the sensor user over a certain period of time, through a seventeenth area 602. The AGP allows daily glucose levels to be checked in a continuous graph. The glucose-related biometric information may include average glucose, the GMI, the coefficient of variation, and whether the sensor user's glucose is in the target range.

Referring to FIG. 6B, when there is a request from a third party other than the healthcare provider who sets the blind mode with regard to a sensor user for whom the blind mode is set, the sensor user's biometric information may not be output, the sensor user's glucose-related biometric information may be blurred and provided through an eighteenth area 611, and the sensor user's AGP may be blurred and provided through a nineteenth area 612.

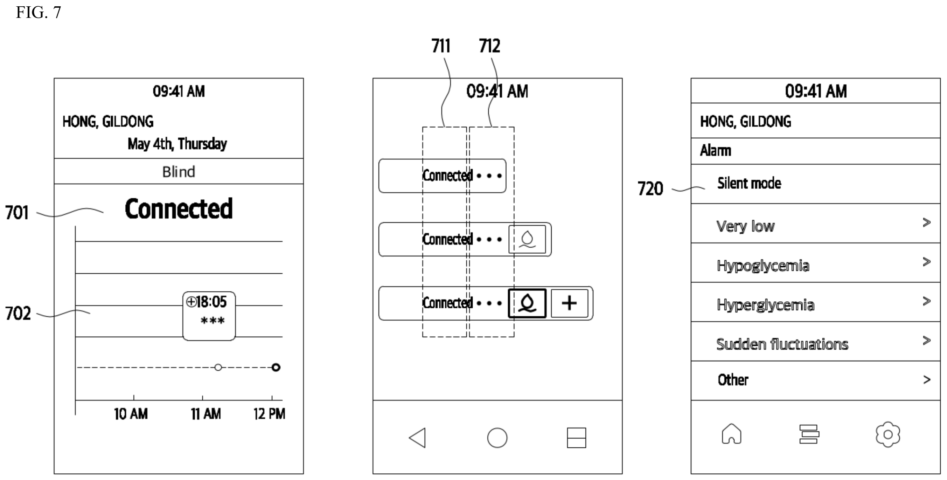

FIG. 7 is a diagram to explain the process by which biometric information of a sensor user under the blind mode is blurred and provided to the sensor user according to an example embodiment.

Referring to FIG. 7, when a sensor user for whom the blind mode is set requests biometric information of himself/herself, the sensor user's biometric information may not be output, and the biometric information of himself/herself may be provided in a blurred form to the sensor user. For example, in an application, through a twentieth area 701, provided is a connection state with the sensor user's terminal and the sensor that can provide biometric information to the sensor user. Further, in the application, the graph may be blurred and presented through a twenty-first area 702. For example, a screen may be showing "***" instead of the CGM value.

A widget corresponding to the application may also provide a screen displaying "***" instead of the connection state via a twenty-second area 711 and the CGM value via a twenty-third area 712.

Further, sensor users may typically set alarms in response to glucose-related biometric information in the application via a twenty-fourth area 720, but for a sensor user for which the blind mode is set, an icon to set an alarm corresponding to glucose-related biometric information through the twenty-fourth area 720 of the application may be deactivated.

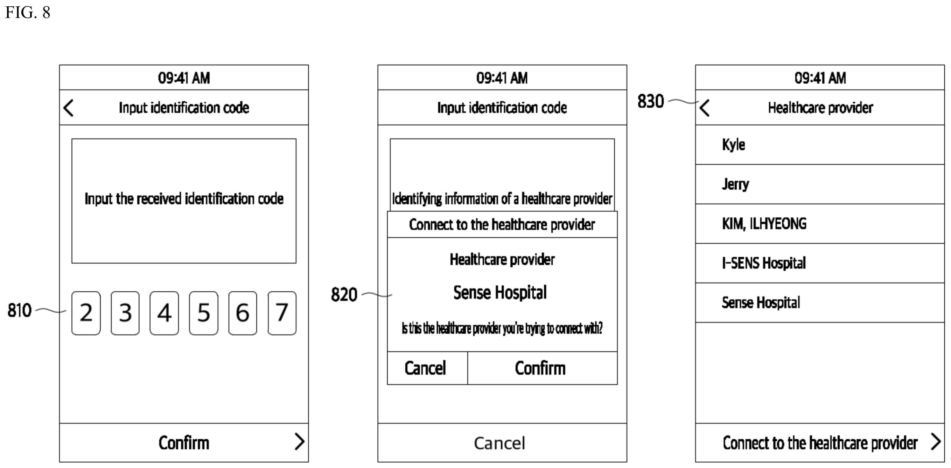

FIG. 8 is a diagram illustrating a process of a sensor user adding a healthcare provider to a blind group through an application according to an example embodiment.

Referring to FIG. 8, a healthcare provider may be added to a blind group that includes a sensor user via a web-generated identification code. For example, when the identification code is entered via a twenty-fifth area 810, an alarm message may be displayed asking to check the healthcare provider corresponding to the identification code via a twenty-sixth area 820, and the healthcare provider corresponding to the identification code may be added to the blind group. The sensor user may check information about healthcare providers connected to the sensor user through a twenty-seventh area 830.

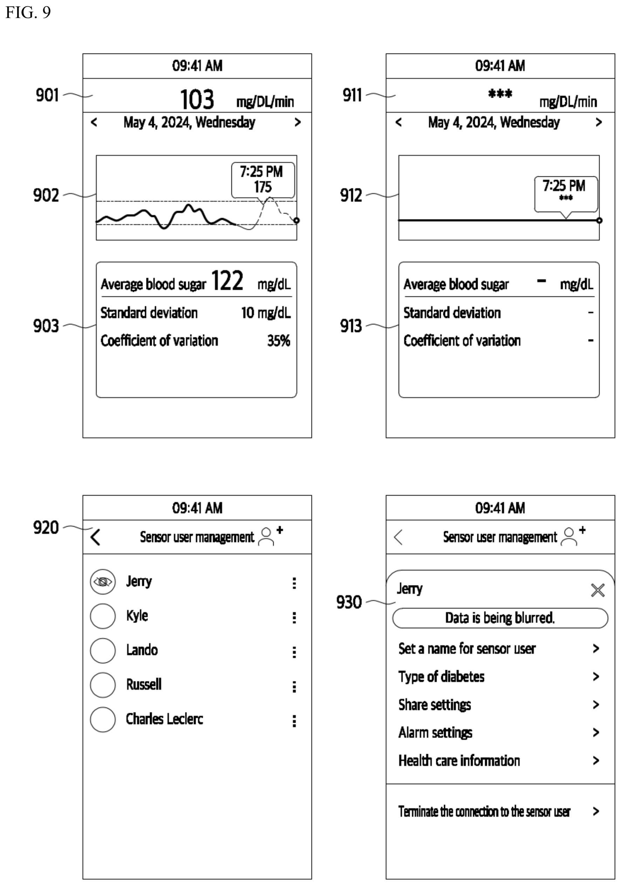

FIG. 9 is a diagram illustrating a process in which biometric information of a sensor user for whom the blind mode is set is identified in an example embodiment.

Referring to FIG. 9, with regard to a sensor user for which the blind mode is set, when a healthcare provider that sets the blind mode for a sensor user requests the sensor user's biometric information, biometric information of the sensor user may be provided. For example, if there is a request from the healthcare provider that set the blind mode for the sensor user, the electronic device 100 may provide CGM values through a twenty-eighth area 901 of the application, glucose trends through a twenty-ninth area 902 and the remaining glucose-related biometric information through a thirtieth area 903.

Meanwhile, with regard to the sensor user for which the blind mode is set, if a healthcare provider other than the healthcare provider that sets the blind mode for the sensor user requests the sensor user's biometric information, the sensor user's biometric information may not be output, or the sensor user's biometric information may be provided in a blurred state. For example, if there is a request from a healthcare provider other than the healthcare provider that set the blind mode to the sensor user, the electronic device 100 may provide a screen with "***" displayed instead of the CGM value via a thirty-first area 911, a screen without the glucose trend activated via the twenty-ninth area 902, and a screen with the remaining glucose-related biometric information not displayed via the thirtieth area 903. Here, the healthcare provider other than the healthcare provider that set the blind mode to the sensor user may identify the sensor user for whom the blind mode is set through a thirty-second area 920 together with the blur icon, and the icon for alarm setting through a thirty-third area 930 may be deactivated for the user.

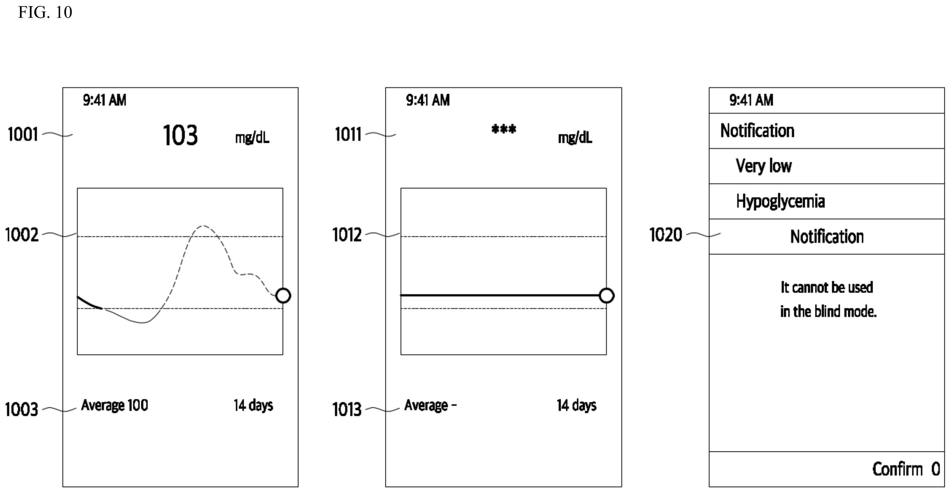

FIG. 10 is a diagram illustrating a process by which a sensor user identifies his/her biometric information according to an example embodiment.

Referring to FIG. 10, a sensor user for whom the blind mode is not set may check the biometric information of himself/herself. For example, in the application, a sensor user may check CGM values through a thirty-fourth area 1001, the glucose trends through a thirty-fifth area 1002, and the average glucose through a thirty-sixth area 1003.

Meanwhile, the sensor user for whom the blind mode is set may not be able to check the biometric information of himself/herself. For example, in the application, a sensor user may check the screen with "***" displayed instead of CGM values through a thirty-seventh area 1011 of the application, the screen where glucose trend is not activated through the thirty-fifth area 1002, and the screen where the average glucose is not provided through the thirty-sixth area 1003. Here, the sensor user may deactivate the screen to set a notification about biometric information via a thirty-eighth area 1020.

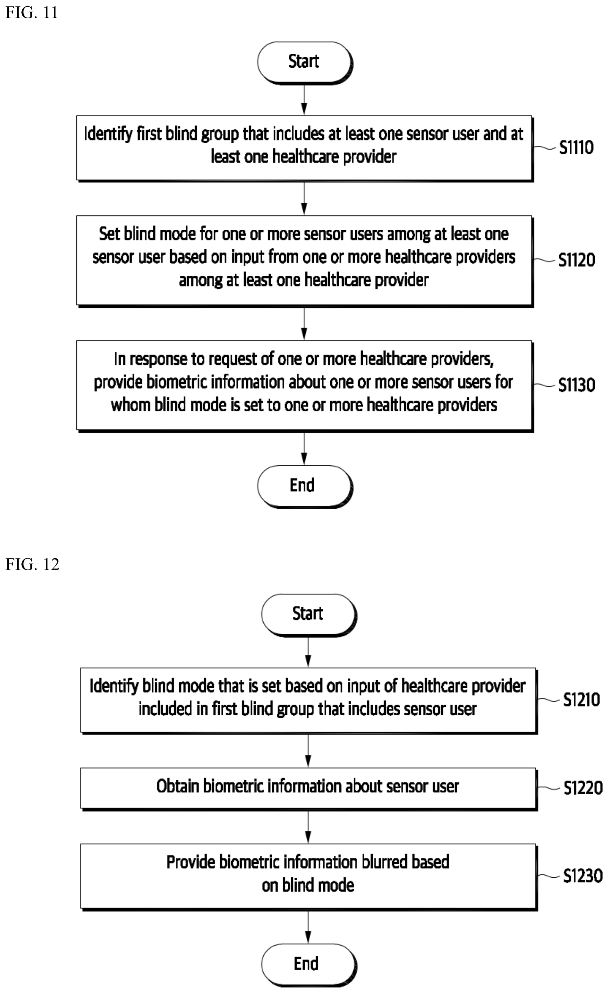

FIG. 11 illustrates a flowchart of a method of operating an electronic device according to an example embodiment.

In operation S1110, the electronic device 100 may identify a first blind group that includes at least one sensor user and at least one healthcare provider. In operation S1120, among at least one healthcare provider, the electronic device 100 may set the blind mode for one or more sensor users among at least one sensor user based on an input from one or more healthcare providers. In operation S1130, in response to a request of the one or more healthcare providers, the electronic device 100 may provide biometric information about one or more sensor users for whom the blind mode is set to one or more healthcare providers.

FIG. 12 illustrates a flowchart of a method of operating a terminal corresponding to a sensor user according to an example embodiment.

In operation S1210, a terminal corresponding to the sensor user may identify the first blind group that includes the sensor user. In operation S1220, the terminal corresponding to the sensor user may identify a blind mode that is set based on an input of the healthcare provider corresponding to the first blind group. In operation S1230, the terminal corresponding to the sensor user may obtain biometric information about the sensor user. In operation S1240, the terminal corresponding to the sensor user may provide biometric information blurred based on the blind mode, and may not output information related to biometric information for one or more sensor users for whom the blind mode is set, even if requested.

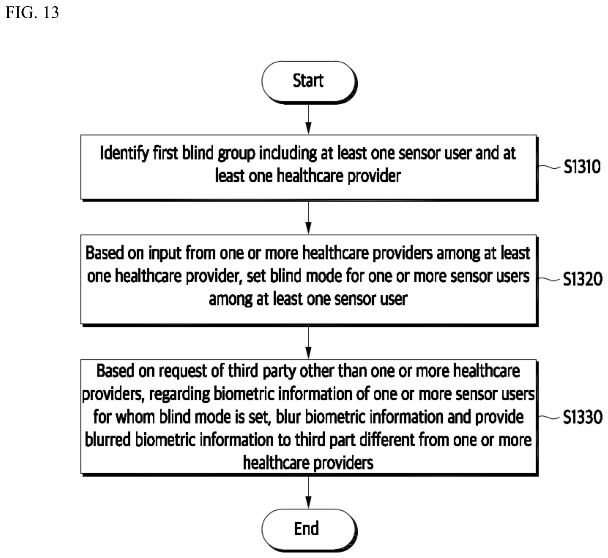

FIG. 13 illustrates a flowchart of a method of operating an electronic device according to an example embodiment.

In operation S1310, the electronic device 100 may identify a first blind group including at least one sensor user and at least one healthcare provider. In operation S1320, based on an input from one or more healthcare providers among at least one healthcare provider, the electronic device 100 may set the blind mode for one or more sensor users among at least one sensor user. In operation S1330, the electronic device 100 may blur and provide biometric information of a sensor user, even at the request of a third party other than the one or more healthcare providers, regarding biometric information of the one or more sensor users for whom the blind mode is set, and may not output information related to biometric information for one or more sensor users for whom the blind mode is set, even if requested.

According to the example embodiments, biometric information of a sensor user may be transmitted in real time only to a healthcare provider who set the blind mode and while the biometric information is set under the blind mode, the sensor user is not provided with his/her biometric information through bypass routes, and thus experimental factors may be controlled more effectively in clinical test or experiment.

## Claims

1. A method of operating an electronic device(100), the method comprising:
identifying (S1110) a first blind group including at least one sensor user and at least one healthcare provider;
based on an input of one or more healthcare providers among the at least one healthcare provider, setting (S1120) a blind mode for one or more sensor users among the at least one sensor user; and
based on a request of the one or more healthcare providers, providing (S1130) the one or more healthcare providers with biometric information on the one or more sensor users for whom the blind mode is set,
wherein, based on a request of a third part other than the one or more healthcare providers, provided is the biometric information blurred for the one or more sensor users for whom the blind mode is set.

2. The method of claim 1, wherein the biometric information comprises at least one biometric information related to glucose.

3. The method of claim 1 or 2, further comprising terminating the blind mode according to an option that is set by the one or more healthcare providers included in the first blind group.

4. The method of any one of the preceding claims, wherein identifying the first blind group comprises adding another sensor user that is different from the at least one sensor user included in the first blind group.

5. The method of claim 4, wherein adding the sensor user comprises using at least one option among:
an option of using an identification code corresponding to the first blind group;
an option of loading a sensor user of another group that is different from the first blind group; and
an option of adding a sensor user that is identified by using an exclusive use account regardless of membership registration for a service that the electronic device provides.

6. The method of claim 5, wherein the option of loading a sensor user of another group that is different from the first blind group corresponds to
loading a sensor user that is connected through a service provided by the one or more healthcare providers and the electronic device among sensor users of the another group that is different from the first blind group.

7. The method of any one of the preceding claims, wherein identifying the first blind group further comprises
deleting one or more sensor users for whom the blind mode is not set in the first blind group, from the first blind group.

8. The method of any one of the preceding claims, further comprising, when no sensor user for which the blind mode is set exists in the first blind group, setting the first blind group as a normal group.

9. The method of any one of the preceding claims, wherein providing the one or more healthcare providers with the biometric information comprises
providing the biometric information in real time by using at least one of a web, an application and a widget.

10. The method of any one of the preceding claims, wherein, in the setting the blind mode,
when a blind mode is set in a second blind group for a sensor user that is also included in the second blind group that is distinguished from the first blind group among the one or more sensor users,
blind mode setting is deactivated in the first blind group for the sensor user that is also included in the second blind group.

11. A method of operating a terminal corresponding to a sensor user, the method comprising:
identifying (S1210) a blind mode that is set based on an input of a healthcare provider included in a first blind group that includes the sensor user;
obtaining (S1220) biometric information of the sensor user; and
providing (S1230) information corresponding to the biometric information blurred based on the blind mode,
wherein the blurred information is provided in response to a request of a third party other than the healthcare provider,
wherein the biometric information comprises at least of biometric information related to glucose.

12. The method of claim 11, further comprising providing the healthcare provider with biometric information that is obtained based on a request of the healthcare provider.

13. The method of claim 11 or 12, further comprising providing the sensor user with information that corresponds to the blurred biometric information regardless of a request of the sensor user.

14. The method of any one of claims 11 to 13, further comprising:
identifying a blind mode that is terminated according to an option that is set by the healthcare provider included in the first blind group; and
providing the biometric information to the sensor user in response to termination of the blind mode.

15. A method of operating an electronic device, the method comprising:
identifying (S1310) a first blind group including at least one sensor user and at least one healthcare provider;
based on an input of one or more healthcare providers among the at least one healthcare provider, setting (S1320) a blind mode for one or more sensor users among the at least one sensor user; and
with respect to biometric information on the one or more sensor users for whom the blind mode is set, based on a request of a third part other than the one or more healthcare providers, blurring (S1330) the biometric information and providing the blurred biometric information to the one or more healthcare providers and the third part that is different from the one or more healthcare providers.
